# EUROPEAN PATENT APPLICATION

(11) **EP 4 851 147 A1**
(43) Date of publication of application: **09.12.2026**
(21) Application number: 25181437.2
(22) Date of filing: 06.06.2025
(51) Int. Cl.: C07D 401/14, A61P 25/00, A61P 25/16, A61P 25/28

(54) **COMPOUNDS FOR THE TREATMENT AND PREVENTION OF DISEASES ASSOCIATED WITH THE AGGREGATION OF ALPHA-SYNUCLEIN**

(71) Applicant: Modag GmbH, 55234 Wendelsheim (DE)
(72) Inventor: The designation of the inventor has not yet been filed
(74) Representative: Vossius & Partner Patentanwälte Rechtsanwälte mbB

(57) **Abstract**

The present invention relates to compounds represented by the general formula la or Ib 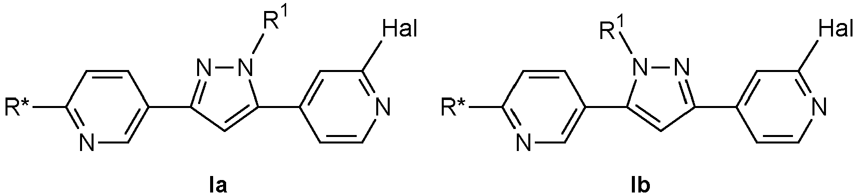 or a prodrug, solvate or pharmaceutically acceptable salt thereof.

These compounds are suitable for treating and preventing diseases which are associated with the aggregation of alpha-synuclein.

## Description

### SUMMARY OF THE INVENTION

The present invention refers to novel compounds which are suitable for treating and preventing diseases which are associated with the aggregation of alpha-synuclein.

### BACKGROUND OF THE INVENTION

A large number of neurological and neurodegenerative diseases are known, many of which are presently not curable. All common neurodegenerative diseases are characterized by the misfolding, aggregation, and deposition of specific proteins in the brain. These diseases include medical conditions such as Parkinson's disease (PD), Dementia with Lewy Bodies (DLB), Multiple System Atrophy (MSA), Alzheimer's disease, progressive supranuclear palsy, corticobasal degeneration, frontotemporal dementia, Creutzfeldt-Jakob disease and many others.

Synucleinopathies are a group of diseases characterized by accumulation and deposition of aggregated and misfolded alpha-synuclein protein (αSYN). Synucleinopathies include Parkinson's disease (PD), Dementia with Lewy Bodies (DLB), and Multiple System Atrophy (MSA). These diseases differ in the distribution of the accumulation and deposition of aggregated and misfolded alpha-synuclein protein in the central and peripheral nervous system. Neuropathologically, they can be distinguished from other neurodegenerative diseases by the disease-specific accumulation and deposition of aggregated and misfolded alpha-synuclein protein, whereas other neurodegenerative diseases are characterized by the accumulation and deposition of other aggregated and misfolded proteins. For example, Alzheimer's disease is characterized by aggregated and misfolded Abeta (Aβ) and tau protein, progressive supranuclear palsy and corticobasal degeneration are characterized by aggregated and misfolded tau protein, also some cases of frontotemporal dementia are characterized by aggregated and misfolded tau protein. Creutzfeldt-Jakob disease is characterized by aggregated and misfolded prion protein.

The disease-specific accumulation and deposition of aggregated and misfolded proteins provides a target both for therapy as well as for diagnosis by compounds which bind to these aggregated and misfolded protein deposits.

WO 2010/000372 relates to a specific compound which is useful in the treatment or prevention of diseases linked to protein aggregation and/or neurodegenerative diseases. The compounds of WO 2010/000372 are particularly suitable for treating diseases linked to protein aggregation including Parkinson's disease. They have been shown to bind to several different aggregated proteins including synuclein, Abeta and tau.

WO 2017/102893 is concerned with water-soluble derivatives of 3,5-diphenyl-diazole compounds, which are effective therapeutic agents for use in treating diseases linked to protein aggregation and/or neurodegenerative diseases. The respective compounds can be efficiently used for oral administration since they have good solubility in water.

WO 2021/099518 refers to compounds which have improved properties as a diagnostic. In particular, the affinity and specificity of binding to alpha-synuclein is improved and the unspecific binding in brain tissue and other tissues is reduced.

Whereas the binding to the intended target is crucial for the therapeutic effect of a compound, there are also additional properties that are relevant for the use of a compound as a therapeutic. These properties include a suitable metabolic stability and low off-target binding because off-target binding can result in unintended side effects.

For emrusolmin, a compound described in WO 2010/000372, it has been observed that exposure levels decrease with multiple dosing compared to a single dose (Reference: Safety, tolerability and pharmacokinetics of the oligomer modulator anle138b with exposure levels sufficient for therapeutic efficacy in a murine Parkinson model: A randomised, double-blind, placebo-controlled phase 1a trial. Levin J, et al. EBioMedicine, 2022 Jun; 80:104021. doi: 10.1016/j.ebiom.2022.104021). This is due to an induction of metabolising enzymes such as CYP1A2 by this compound. CYP1A2 can be upregulated via the AhR (aryl hydrocarbon receptor) pathway. Modulating cytochrome P450 enzymes such as CYP1A2 can affect the pharmacokinetics of co-administered drugs, leading to undesired drug-drug interactions. Moreover, AhR activation can also result in further off-target effects such as metabolic changes and modulation of the immune system. Thus, the induction of CYP1A2 that was observed for emrusolmin and other compounds described in WO 2021/099518 is an undesired property and compounds that have at least the same target binding affinity as emrusolmin but reduced or no CYP1A2 induction would provide a relevant improvement. This is especially important for therapeutic compounds that are dosed for longer periods of time.

At the same time, it is important that therapeutic compounds have a suitable metabolic stability, so that the metabolism and excretion in the body are not too fast.

In view of the foregoing, there was a need for compounds with a low AhR activation, ideally none, and that at the same time show suitable metabolic stability.

### DETAILED DESCRIPTION OF THE INVENTION

The present invention relates to a compound represented by the general formula la or Ib

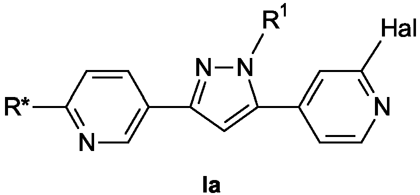

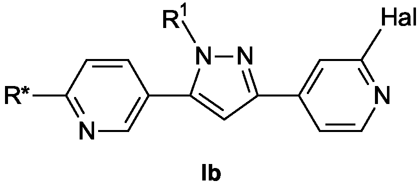

**R*** is selected from

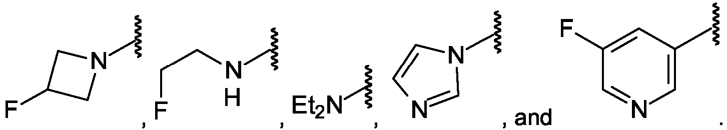

Preferably **R*** is selected from 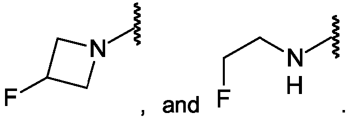 In one embodiment, **R*** 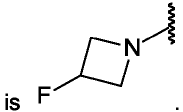 In another embodiment R* is

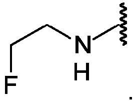

**R¹** is independently selected from hydrogen, C₁₋₄ alkyl and -(CH₂)-O-P(=O)(OR)(OR), wherein C₁₋₄ alkyl can be optionally substituted by one or more halogen. In a preferred embodiment, R¹ is hydrogen, methyl or 2-fluoroethyl. More preferably R¹ is hydrogen.

**R** is hydrogen or a cation. The cation can be any cation that is pharmaceutically acceptable. Preferably, the cation is a monovalent cation. Examples are sodium, lithium, potassium, ammonium and protonated forms of ethanolamine, choline, lysine, meglumine, piperazine, and tromethamine. Preferably, the cation is sodium. In the compounds of the present invention both R can be hydrogen, both R can be cations (the same or different cations), or one R can be hydrogen and the other one can be a cation. Preferably, both R are sodium. Bivalent cations such as Ca²⁺, Mg²⁺, and Zn²⁺ or trivalent cations such as Al³⁺ are possible but not preferred, as the resulting pharmaceutically acceptable salts are less water-soluble. Compounds in which R¹ is -(CH₂)-O-P(=O)(OR)(OR) and their synthesis are described in WO 2017/102893, which is incorporated herein by reference.

**Hal** is halogen. Preferably Hal is selected from Br, Cl and F, more preferably Hal is Cl.

It has been surprisingly found that compounds with general formula la or Ib have only low to no CYP1A2 induction activity and at the same time have at least the binding affinity for alpha-synuclein aggregates of emrusomin and a suitable metabolic stability.

The compounds of the present invention can also be present in the form of prodrugs, solvates or pharmaceutically acceptable salts thereof.

The compounds of the present invention form pharmaceutically acceptable salts which are also within the scope of this invention. Reference to a compound of the present invention herein is understood to include reference to pharmaceutically acceptable salts thereof, unless otherwise indicated. Pharmaceutically acceptable (i.e., non-toxic, physiologically acceptable) pharmaceutically acceptable salts are preferred, although other pharmaceutically acceptable salts are also useful, e.g., in isolation or purification steps which may be employed during preparation or in *in vitro* methods. Pharmaceutically acceptable salts of the compounds of the present invention may be formed, for example, by reacting a compound with an amount of acid, such as an equivalent amount, in a medium such as one in which the pharmaceutically acceptable salt precipitates or in an aqueous medium followed by lyophilization.

The compounds which contain a basic moiety may form pharmaceutically acceptable salts with a variety of organic and inorganic acids. Exemplary acid addition pharmaceutically acceptable salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, hydroxyethanesulfonates (e.g., 2-hydroxyethanesulfonates), lactates, maleates, methanesulfonates, naphthalenesulfonates (e.g., 2-naphthalenesulfonates), nicotinates, nitrates, oxalates, pectinates, persulfates, phenylpropionates (e.g., 3-phenylpropionates), phosphates, picrates, pivalates, propionates, salicylates, succinates, sulfates (e.g., those formed with sulfuric acid), sulfonates (e.g., those mentioned herein), tartrates, thiocyanates, toluenesulfonates such as tosylates, undecanoates, and the like.

Prodrugs and solvates of the compounds of the invention are also contemplated herein. The term "prodrug" as employed herein denotes a compound which, upon administration to a subject, undergoes chemical conversion by metabolic or chemical processes to yield a compound of the present invention or a pharmaceutically acceptable salt and/or solvate thereof.

Solvates of the compounds of the present invention include, for example, hydrates.

All stereoisomers of the present compounds (e.g., those which may exist due to asymmetric carbons on various substituents), including enantiomeric forms and diastereomeric forms, are contemplated within the scope of this invention. Individual stereoisomers of the compounds of the invention may, for example, be substantially free of other isomers (e.g., as a pure or substantially pure optical isomer having a specified activity), or may be admixed, for example, as racemates or with all other, or other selected, stereoisomers. The chiral centers of the compounds of the present invention may have the S- or R-configuration as defined by the IUPAC 1974 Recommendations.

The racemic forms can be resolved by physical methods, such as fractional crystallization, separation or crystallization of diastereomeric derivatives or separation by chiral column chromatography. The individual optical isomers can be obtained from the racemates by any suitable method, including without limitation, pharmaceutically acceptable salt formation with an optically active acid followed by crystallization.

All configurational isomers of the compounds of the present invention are contemplated, either in admixture or in pure or substantially pure form. The definition of compounds of the present invention embraces both cis (Z) and trans (E) alkene isomers, as well as cis and trans isomers of cyclic hydrocarbon or heterocyclic rings.

Deuterated versions of the claimed compounds can also be provided. The position at which the deuteration is present is not particularly limited.

The compounds of the present invention can be provided in the form of a pharmaceutical composition which optionally includes a pharmaceutically acceptable carrier or excipient.

The present invention is directed to a compound of the present invention or a prodrug, solvate or pharmaceutically acceptable salt thereof for the use in the treatment or prevention of a disease linked to the aggregation of alpha-synuclein.

In a further embodiment, the present invention relates to a method of treating or preventing a disease linked to the aggregation of alpha-synuclein in which a therapeutically effective amount of a compound of the present invention or a prodrug, solvate or pharmaceutically acceptable salt thereof is administered to a patient in need thereof.

In another embodiment, the present invention refers to the use of a compound of the present invention or a prodrug, solvate or pharmaceutically acceptable salt thereof for the manufacture of a medicament for treating or preventing a disease linked to the aggregation of alpha-synuclein.

Further embodiments are the use of a compound of the present invention for the preparation of a pharmaceutical composition for treating or preventing a disease linked to the aggregation of alpha-synuclein as well as a method of treating or preventing a disease linked to the aggregation of alpha-synuclein comprising administering a therapeutically effective amount of a compound of the present invention to a patient in need thereof. This includes the application of the compound as a "pharmaceutical composition" as described below.

The term "aggregation", in accordance with the present invention, refers to the formation of oligomeric or multimeric complexes of alpha-synuclein, which may be accompanied by the integration of additional biomolecules, like carbohydrates, nucleic acids, lipids and/or metal ions, into the complexes.

The term "disease linked to aggregation of alpha-synuclein", as used herein, refers to those diseases which are characterized by the presence of aggregated alpha-synuclein, either as the disease-defining pathology or as a co-pathology. Such aggregated alpha-synuclein may form deposits in specific tissue, more preferably in nerve tissue or tissue of the brain. The extent of aggregation depends on the particular disease. The disease linked to alpha-synuclein aggregation is not particularly limited and is typically selected from Parkinson's disease, dementia with Lewy bodies, multiple system atrophy, Alzheimer's disease, progressive supranuclear palsy, and frontotemporal dementia, preferably Parkinson's disease, dementia with Lewy bodies, and multiple system atrophy.

As used herein, the term "therapeutically effective amount" refers to an amount sufficient to elicit the desired biological response. In the present invention, the desired biological response is the inhibition of alpha-synuclein aggregation and/or the reduction in the amount of aggregated alpha-synuclein present in the tissue.

In accordance with the present invention, the term "pharmaceutical composition" relates to a composition for administration to a patient, preferably a mammal, more preferably a human patient. The pharmaceutical composition of the invention comprises the compounds recited above and optionally further molecules capable of altering the characteristics of the compounds of the invention thereby, for example, stabilizing, modulating and/or activating their function. The composition may be in solid, liquid or gaseous form and may be, inter alia, in the form of (a) powder(s), (a) tablet(s), (a) solution(s) or (an) aerosol(s). The pharmaceutical composition of the present invention may, optionally and additionally, comprise a pharmaceutically acceptable carrier or excipient. Examples of suitable pharmaceutical carriers and excipients are well-known in the art and include phosphate buffered saline solutions, water, emulsions, such as oil/water emulsions, various types of wetting agents, sterile solutions, organic solvents including DMSO etc. Compositions comprising such carriers can be formulated by well-known conventional methods. Possible pharmaceutical compositions are disclosed in WO 2023/166045.

The pharmaceutical composition will be formulated and dosed in a fashion consistent with good medical practice, taking into account the clinical condition of the individual patient, the site of delivery of the pharmaceutical composition, the method of administration, the scheduling of administration, and other factors known to practitioners. The "therapeutically effective amount" of the pharmaceutical composition for purposes herein is thus determined by such considerations. The skilled person knows that the therapeutically effective amount of pharmaceutical compositions administered to an individual will, inter alia, depend on the nature of the compound.

Pharmaceutical compositions of the invention may be administered orally, rectally, parenterally, intracisternally, intravaginally, intraperitoneally, topically (as by powders, ointments, drops or transdermal patch), bucally, or as an oral or nasal spray. Preferably, they will be administered orally.

By "pharmaceutically acceptable carrier" is meant a non-toxic solid, semisolid or liquid filler, diluent, encapsulating material or formulation auxiliary of any type.

The term "parenteral" as used herein refers to modes of administration which include intravenous, intramuscular, intraperitoneal, intrasternal, subcutaneous and intraarticular injection and infusion.

For therapeutic purposes, the pharmaceutical composition can also be suitably administered by sustained release systems. Suitable examples of sustained-release compositions include semi-permeable polymer matrices in the form of shaped articles, e.g., films, or microcapsules. Sustained-release matrices include polylactides (U.S. Pat. No. 3,773,919, EP 0 058 481), copolymers of L-glutamic acid and gamma-ethyl-L-glutamate (Sidman, U. et al., Biopolymers 22:547-556 (1983)), poly (2-hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D-(-)-3-hydroxybutyric acid (EP 133 988). Sustained release pharmaceutical compositions can also include liposomally entrapped compounds. Liposomes containing the pharmaceutical composition are prepared by methods known per se: DE 32 18 121; Epstein et al., Proc. Natl. Acad. Sci. (USA) 82:3688-3692 (1985); Hwang et al., Proc. Natl. Acad. Sci. (USA) 77:4030-4034 (1980); EP 52 322; EP 36 676; EP 88 046; EP 143 949; EP 142 641; Japanese Pat. Appl. 83-118008; U.S. Pat. Nos. 4,485,045 and 4,544,545; and EP 102324. Ordinarily, the liposomes are of the small (about 200 - 800 Angstroms) unilamellar type in which the lipid content is greater than about 30 mol% cholesterol, the selected proportion being adjusted for the optimal therapy.

For parenteral administration, the pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Preferably, the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their pharmaceutically acceptable salts; antioxidants such as ascorbic acid; low molecular weight (less than about ten residues) (poly)peptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 µm membranes). Therapeutic components of the pharmaceutical composition generally are placed into a container having a sterile access port, for example, an intravenous solution bag or vial having a stopper pierceable by a hypodermic injection needle.

The components of the pharmaceutical composition ordinarily will be stored in unit or multidose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic Water-for-Injection.

The present invention also relates to the use of a compound as defined above for inhibiting alpha-synuclein aggregation *in vitro* or *ex vivo.*

The following examples are intended to illustrate the invention. However, they are not to be construed as limiting.

### EXAMPLES

### Example 1: Synthesis

### Chemical synthetic procedures

The following methods are presented with details as to the preparation of a compound of the invention and the illustrative examples. A compound of the invention can be prepared from known or commercially available starting materials and reagents by one skilled in the art of organic synthesis.

All starting materials and solvents were of commercial grade and were used as received unless noted otherwise.

**Thin layer chromatography (TLC)** was conducted using Macherey-Nagel precoated sheets, 0.25 mm ALUGRAM^{®} SIL G/UV254 plates, detection with UV and/or by charring with 10 wt% ethanolic phosphomolybdic acid reagent followed by heating at 200 °C.

**Flash column chromatography** was performed using Merck silica gel 60 (0.063 to 0.100 mm).

**Analytical high performance liquid chromatography (HPLC)** was performed using a Waters HPLC system with a Waters 996 Photodiode Array Detector. All separations involved a mobile phase of 0.1% trifluoroacetic acid (TFA) (v/v) in water and 0.1% TFA in acetonitrile. HPLC was performed using a reversed-phase (RP) column Eurospher RP 18, 100 Å, 5 µm, 250×4.6 mm at flow rate of 1 mL·min⁻¹.

**Electrospray ionization mass spectrometry (ESI-MS)** and **liquid chromatography/ mass spectrometry (LC/MS)** analyses were obtained using a Waters Micromass ZQ 4000 mass spectrometer in conjunction with the Waters HPLC apparatus described above.

**NMR spectra** were recorded using a 400 MHz Bruker Avance spectrometer (Bruker AG, Rheinstetten, Germany) equipped with a TXI HCN z-gradient probe. All spectra were processed using TOPSPIN 3.1 (Bruker AG, Karlsruhe, Germany). ¹H NMR chemical shifts (δ) are reported in parts per million (ppm) relative to CHCl₃ and DMSO-d5 as internal standards. Data are reported as follows: chemical shift, multiplicity (s = singlet, d = doublet, t = triplet, q = quartet, qi = quintet, dd = doublet of doublets, dt = doublet of triplets, b = broadened, m = multiplet), coupling constants (J, given in Hz), integration.

### Synthesis of starting materials

### tert-Butyl (5-acetylpyridin-2-yl)carbamate

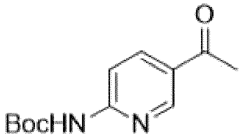

t*ert*-Butyl (5-acetylpyridin-2-yl)carbamate was prepared according to a previously reported method (ACS Med. Chem. Lett. 2022, 13, 1510).

### tert-Butyl (5-acetylpyridin-2-yl)(2-fluoroethyl)carbamate

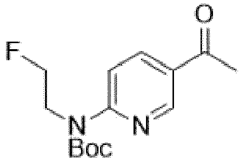

A mixture of of *tert*-butyl (5-acetylpyridin-2-yl)carbamate (1.18 g, 5 mmol) and Cs₂CO₃ (2.61 g, 8 mmol) in DMSO (10 mL) was stirred at room temperature for 10 min. 1-Fluoro-2-iodoethane (640 µL, 1.39 g, 8 mmol) was added and stirring was continued for 3 h. The reaction mixture was quenched with 0.5M phosphate buffer pH 7 (20 mL), ethyl acetate (20 mL) was added, the organic phase was separated, the mixture was washed with water, brine, dried over Na₂SO₄ and evaporated. The crude product was purified by flash chromatography on silica gel (*n*-hexane/EtOAc = 3/1, R_{f} = 0.54) to give the title product (960 mg, 3.4 mmol, 68%) as a yellow oil.

¹H NMR (400 MHz, DMSO-d6) *δ* = 8.93 (d, *J* = 1.8 Hz, 1H), 8.23 (dd, *J* = 8.8, 2.4 Hz, 1H), 7.82 (dd, *J* = 8.8, 0.5 Hz, 1H), 4.63 (dt, *J* = 47.3, 5.3 Hz, 2H), 4.31 (dt, *J* = 22.8, 5.3 Hz, 2H), 2.58 (s, 3H), 1.49 (s, 9H). LC-MS: sample 0.5 mg/mL in CH₃CN; 10 µL injection volume; column: Eurospher RP18 100Å, 5µm, 250×4.6 mm, solvents: water (+0.1% TFA, A) and CH₃CN (+0.1% TFA, B), gradient: B 5% → 100% in 30 min, detector UV 260 nm, peak RT 23.6 min, mass 227.2 (100%) [M+H-C₄H₈]⁺.

### 1-[6-(Diethylamino)pyridin-3-yl]ethanone

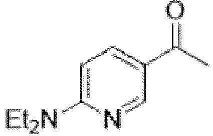

1-[6-(Diethylamino)pyridin-3-yl]ethanone was prepared according to a previously reported method (Bioorg. Med. Chem Lett. 2023, 92, 129385).

### 1-[6-(3-Fluoroazetidin-1-yl)pyridin-3-yl]ethanone

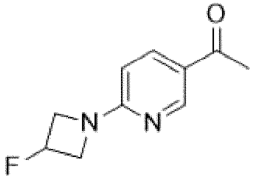

1-[6-(3-Fluoroazetidin-1-yl)pyridin-3-yl]ethanone was prepared according to a published protocol (WO 2011/071570 A1) using 1-(6-chloropyridin-3-yl)ethanone and 3-fluoroazetidine hydrochloride as starting materials. A mixture of 1-(6-chloropyridin-3-yl)ethanone (3.11 g, 20 mmol), 3-fluoroazetidine hydrochloride (3.35 g, 30 mmol), pyridine (2.42 mL, 2.37 g, 30 mmol), Cs₂CO₃ (7.17 g, 22 mmol) and DMSO (20 mL) were placed in a sealed pressure tube, flushed with argon, and stirred at 120 °C. The reaction progress was controlled by LC-MS. For this, the mixture was cooled, a 50 µL aliquot was sampled, the reaction was quenched with 5% aqueous citric acid, extraction was performed with EtOAc, the solvent was removed with nitrogen flow, the residue was dissolved in CH₃CN and controlled by LC-MS. After 15 h, an almost full conversion of educt was observed. The reaction mixture was cooled, poured into 200 mL of 0.5M phosphate buffer pH 7 and extracted with EtOAc (3×50 mL). The combined organic layers were washed with brine, dried over Na₂SO₄ and concentrated *in vacuo.* The residue was evaporated once with toluene (50 mL) to remove the traces of pyridine to give an oily crude product (4.16 g). It was purified by flash chromatography on silica gel (CHCl₃/MeOH = 100/1, R_{f} = 0.35) to give the title product (2.2 g, 11.3 mmol, 57%) as a brownish solid.

¹H NMR (400 MHz, CDCl₃) *δ* = 8.75 (d, *J* = 1.8 Hz, 1H), 8.04 (dd, *J* = 8.8, 2.3 Hz, 1H), 6.29 (dd, *J* = 8.8, 1.1 Hz, 1H), 5.47 (ddd, *J* = 59.9, 18.8, 6.7 Hz, 1H), 4.47-4.37 (m, 2H), 4.30-4.20 (m, 2H), 2.51 (s, 3H). LC-MS: sample 0.5 mg/mL in CH₃CN; 10 µL injection volume; column: Eurospher RP18 100Å, 5µm, 250×4.6 mm, solvents: water (+0.1% TFA, A) and CH₃CN (+0.1% TFA, B), gradient: B 5% → 100% in 30 min, detector UV 260 nm, peak RT 9.2 min, mass 195.1 (100%) [M+H]⁺.

### 1-[6-(1H-Imidazol-1-yl)pyridin-3-yl]ethanone

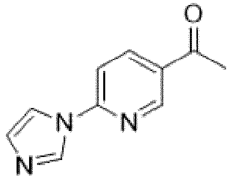

1-[6-(1*H*-Imidazol-1-yl)pyridin-3-yl]ethanone was prepared according to a previously reported method *(*Bioorg. Med. Chem Lett. 2011, 21, 5849).

### 2-Fluoropyridine-4-carbonyl chloride

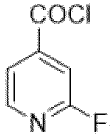

2-Fluoropyridine-4-carbonyl chloride was prepared according to a previously reported method (J. Med. Chem. 1990, 33, 1667).

### 1-(5'-Fluoro-2,3'-bipyridin-5-yl)ethanone

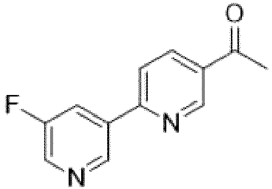

1-(5'-Fluoro-2,3'-bipyridin-5-yl)ethanone was prepared according to a published protocol (Bioorg. Med. Chem Lett. 2010, 18, 3860) using 1-(6-bromopyridin-3-yl)ethanone and (5-fluoropyridin-3-yl)boronic acid as starting materials. A mixture of 1-(6-bromopyridin-3-yl)ethanone (10 mmol, 2.00 g), (5-fluoropyridin-3-yl)boronic acid (11 mmol, 1.55 g), Na₂CO₃ (7 mmol, 742 mg) and PdCl₂[Ph₃P]₂ (0.3 mmol, 211 mg) was placed in a mixture of CH₃CN and water (1/1, 80 mL). N₂ gas was bubbled into the mixture for 10 min, and the mixture was stirred in a sealed tube at 80 °C for 24 h. The cooled mixture was concentrated, the residue was extracted with CH₂Cl₂ (2×50 mL). The combined organic phases were dried over MgSO₄, filtered through a plug of Al₂O₃ activity I, and concentrated *in vacuo.* The crude product (2.12 g) was crystallized from EtOH (20 mL) to give the title product (1.73 g, 8.0 mmol, 80%) as a beige solid.

¹H NMR (400 MHz, CDCl₃) δ = 9.26 (d, *J* = 1.8 Hz, 1H), 9.07 (s, 1H), 8.57 (d, *J* = 2.7 Hz, 1H), 8.35 (dd, *J* = 8.3, 2.2 Hz, 1H), 8.17 (dt, *J* = 9.5, 2.2 Hz, 1H), 7.89 (d, *J* = 8.3 Hz, 1H), 2.69 (s, 3H). LC-MS: sample 0.5 mg/mL in CH₃CN; 10 µL injection volume; column: Eurospher RP18 100Å, 5µm, 250×4.6 mm, solvents: water (+0.1% TFA, A) and CH₃CN (+0.1% TFA, B), gradient: B 5% → 100% in 30 min, detector UV 260 nm, peak RT 18.3 min, mass 217.1 (100%) [M+H]⁺.

### Synthesis of compounds

The following compounds were prepared according to a published protocol (WO 2021/099518): **M204, M225 and M255.**

### Method A: Synthesis of 1H-pyrazoles

Those skilled in the art will recognize that the compound **M269A** and compound **M269B** depicted below are two tautomeric forms of the same compound. All such tautomeric forms are considered as part of this invention. As an illustration, all of the tautomeric forms of the pyrazole moiety as depicted, for example, for the compound **M269** below are included in this invention. In a related manner, those skilled in the art will recognize that the compound names contained herein are based on a nomenclature convention wherein the tautomer configuration is depicted as with respect to compound **M269A** below. Thus, the diethylamino substituent is in the three position. An alternative nomenclature convention would be based on the tautomer compound **M269B** below and in that convention the diethylamino substituent is in the five position.

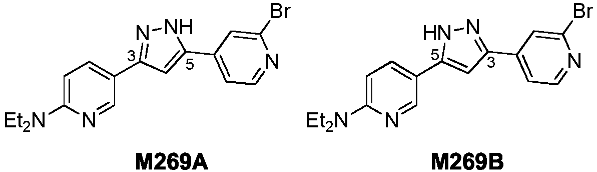

### 5-[5-(2-Bromopyridin-4-yl)-1H-pyrazol-3-yl]-N,N-diethylpyridin-2-amine M269

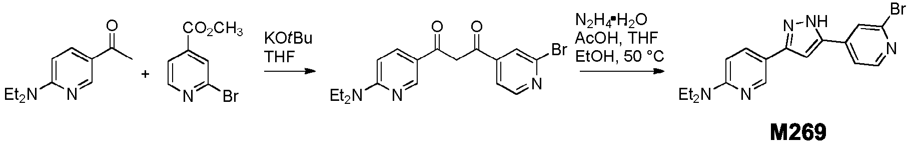

A solution of potassium tert-butoxide (281 mg, 2.5 mmol) in dry THF (5 mL) was added under nitrogen to a stirred solution of 1-[6-(diethylamino)pyridin-3-yl]ethanone (384 mg, 2.0 mmol) and methyl 2-bromopyridine-4-carboxylate (540 mg, 2.5 mmol) in THF (8 mL). The reaction mixture was stirred for 24 h. A 10 µL aliquot was sampled, quenched with 0.5M phosphate buffer pH 7, extracted with EtOAc and analyzed by TLC. A full conversion of ketone was observed. To the mixture EtOH (2 mL), AcOH (600 µL, 630 mg, 10.5 mmol) and hydrazine monohydrate (500 µL, 500 mg, 10 mmol) were added. After stirring at 50 °C for 24 h the reaction mixture was cooled to room temperature and concentrated *in vacuo,* 0.5M phosphate buffer pH 7 (30 mL) was added, the resulting solid was filtered off, washed with water and air-dried giving the crude product (575 mg) as a brown solid. Crystallization from *n*BuOH (5 mL) afforded the title product (350 mg, 0.94 mmol, 47% over 2 steps) as a white solid.

### Method B: Removing of Boc protective group

### 5-[5-(2-Bromopyridin-4-yl)-1H-pyrazol-3-yl]-N-(2-fluoroethyl)pyridin-2-amine M268

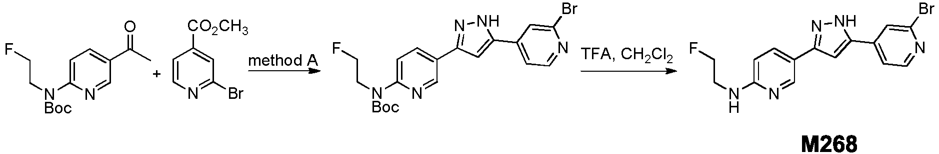

Trifluoroacetic acid (1 mL, 1.48 g, 13 mmol) was added to a solution of {5-[5-(2-bromopyridin-4-yl)-1*H*-pyrazol-3-yl]pyridin-2-yl}(2-fluoroethyl)carbamate (300 mg, 0.65 mmol) in CH₂Cl₂ (20 mL), prepared from *tert*-butyl (5-acetylpyridin-2-yl)(2-fluoroethyl)carbamate (576 mg, 2.0 mmol) and methyl 2-bromopyridine-4-carboxylate (540 mg, 2.5 mmol) according to Method A. The mixture was stirred at room temperature for 5 h and concentrated *in vacuo,* then water (10 mL) was added followed by 1N NaOH aqueous solution (pH-12). The product was extracted with CHCl₃ (3×10 mL), the combined organic phases were dried over Na₂SO₄ and concentrated. The residue was purified by flash chromatography on silica gel (*n-*hexane/EtOAc = 1/1, loading in EtOAc). Yield 70 mg (0.19 mmol, 10% over 3 steps).

### Method C: Synthesis of 1H-pyrazoles

### 2-Fluoro-4-{3-[6-(3-fluoroazetidin-1-yl)pyridin-3-yl]-1H-pyrazol-5-yl}pyridine M279

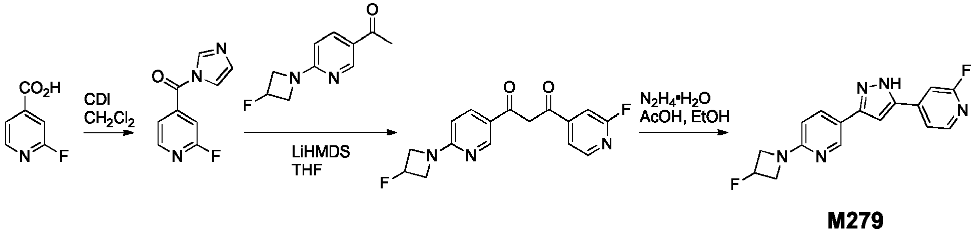

### Step 1

1,1'-Carbonyldiimidazole (1.78 g, 11 mmol) was added in portions to a stirred suspension of 2-fluoropyridine-4-carboxylic acid (1.41g, 10 mmol) in CH₂Cl₂ (20 mL). The resulting mixture was stirred at room temperature for 15 h, then washed with 0.5M phosphate buffer pH 6.5 (15 mL), and water (15 mL), dried over Na₂SO₄ and concentrated *in vacuo* to give 1.4 g (7.3 mmol, 73%) of product as a white solid which was used without further purification.

### Step 2

In a 50 mL Schlenk flask with septum 1-[6-(1*H*-imidazol-1-yl)pyridin-3-yl]ethanone (388 mg, 2 mmol) was dissolved in THF (10 mL) under nitrogen. LiHMDS (1M in THF, 2.1 mL, 2.1 mmol) was added quickly via a syringe with stirring. After 5 min pure (2-fluororyridin-4-yl)(1*H*-imidazol-1-yl)methanone (382 mg, 2 mmol) was added in one portion, and the mixture was stirred for 30 min. To the mixture EtOH (5 mL), AcOH (2 mL, 2.1 g, 35 mmol) and hydrazine monohydrate (2 mL, 2.06 g, 41 mmol) were added, the mixture was stirred at room temperature for 6 h and concentrated *in vacuo.* The residue was quenched with 0.5M phosphate buffer pH 7 (50 mL), the precipitated solid was filtered off, washed with water and air-dried to give the crude product (417 mg). It was dissolved in trifluoroethanol (TFE, 20 mL), and applied to 3 g silica gel. TFE was evaporated; the applied product was purified by flash chromatography (Al₂O₃ activity II, CHCl₃) to give 397 mg of solid. It was triturated with CH₃CN, filtered off and air-dried to afford 303 mg (0.97 mmol, 48%) of pure product as a white solid.

### Method D: Synthesis of 1H-pyrazoles

### 2-Fluoro-4-{3-[6-(1H-imidazol-1-yl)pyridin-3-yl]-1H-pyrazol-5-yl}pyridine M286

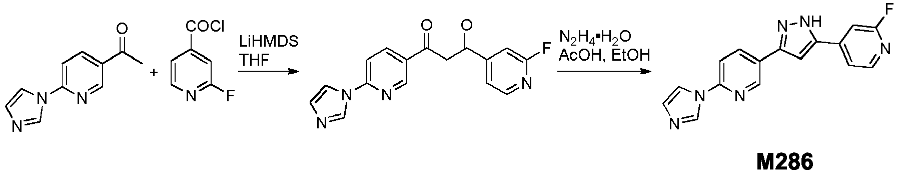

In a 50 mL Schlenk flask with septum 1-[6-(1*H*-imidazol-1-yl)pyridin-3-yl]ethanone (374 mg, 2 mmol) was suspended in THF (20 mL) under nitrogen. LiHMDS (1M in THF, 2.1 mL, 2.1 mmol) was added quickly via a syringe with stirring. After 2 min, pure 2-fluoropyridine-4-carbonyl chloride (160 mg, 1 mmol) was added in one portion, and the mixture was stirred for 10 min. To the mixture, EtOH (5 mL), AcOH (2 mL, 2.1 g, 35 mmol) and hydrazine monohydrate (2 mL, 2.06 g, 41 mmol) were added. The mixture was stirred at room temperature for 15 h and concentrated *in vacuo.* The residue was quenched with 0.5M phosphate buffer pH 7 (20 mL), the precipitated solid was filtered off, washed with water and air-dried to give the crude product (680 mg). It was dissolved in trifluoroethanol (TFE, 20 mL), filtered (0.22 µm PTFE syringe filter) and applied to 4 g silica gel. TFE was evaporated, the applied product was purified by flash chromatography (SiO₂, CHCl₃/MeOH = 95/5) to give 250 mg of crude product. It was dissolved in TFE (15 mL) and HPLC purified with the column Eurospher RP18 100Å, 5µm, 250×16 mm using solvents: water (+0.1% TFA, A) and CH₃CN (+0.1% TFA, B), gradient: B 20% → 50% in 30 min. The combined fraction containing the product was concentrated, the residue was stirred with 1M phosphate buffer pH 7 (10 mL) for 1 h, the solid was filtered off, washed with water and air-dried to give the pure compound (126 mg, 0.41 mmol, 41%).

### Method E: Synthesis of 1H-pyrazoles

### 5-[5-(2-Chloropyridin-4-yl)-1H-pyrazol-3-yl]-N-(2-fluoroethyl)pyridin-2-amine M277

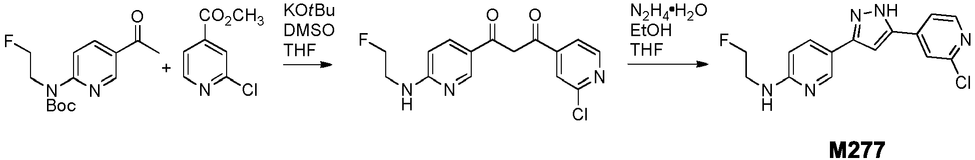

To a solution of methyl 2-chloroisonicotinate (257 mg, 1.5 mmol) and *tert*-butyl (5-acetylpyridin-2-yl)(2-fluoroethyl)carbamate (282 mg, 1 mmol) in dry DMSO (3 mL) a solution of *t*BuOK (224 mg, 2 mmol) in dry THF (4 mL) was added dropwise in 5 min at room temperature. The red dark mixture was stirred at room temperature for 2 hours. The reaction was quenched with phosphate buffer (0.25 M, pH 7, 25 mL) and extracted twice with ethyl acetate (30 mL and 20 mL). Combined organic fractions were washed with brine, dried over Na₂SO₄ and solvents were evaporated *in vacuo.* The crude residue was purified by flash chromatography on silicagel (25 g, DCM → DCM/MeOH = 100 → 100/2) to provide 1-(2-chloropyridin-4-yl)-3-{6-[(2-fluoroethyl)amino]pyridin-3-yl}propane-1,3-dione (130 mg, 40%) as a yellow solid. The mixture of 1-(2-chloropyridin-4-yl)-3-{6-[(2-fluoroethyl)amino]pyridin-3-yl}propane-1,3-dione (130 mg, 0.4 mmol) and hydrazine hydrate (50 mg, 1 mmol) in THF (4 mL) and ethanol (1 mL) was stirred at room temperature for 20 hours. The solvents were evaporated *in vacuo* and the residue was triturated in 10 mL of water followed by sonication for 2 minutes in an ultrasonic bath. The suspension was centrifuged down, the supernatant was removed and the pellets were washed with water (2 ml). After a second centrifugation, the pellets were resuspended in water (2 mL) and lyophilized. The lyophilized residue was purified by flash chromatograpy on silica gel (20 g, CHCl₃/MeOH = 10/1) to provide 5-[5-(2-chloropyridin-4-yl)-1*H*-pyrazol-3-yl]-*N-*(2-fluoroethyl)pyridin-2-amine (90 mg, 28% over two steps) as a yellow solid.

Exemplary compounds of the present invention are shown in Tables 1 and 2. Table 1 shows the name, structure, IUPAC name, starting materials for the preparation, method of synthesis and the chemical yield of a particular example compound. Table 2 shows the High Performance Liquid Chromatography (HPLC) retention time, molecular weight found using Low Resolution Mass Spectrometry coupled with HPLC, and ¹H Nuclear Magnetic Resonance (¹H-NMR) for a particular example compound.

**Table 1**

| **No.** | **Structure** | **IUPAC** | **Reagents** | **Mtd./ Yield (%)** |
|---|---|---|---|---|
| M268 | 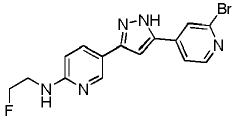 | 5-[5-(2-bromopyridin -4-yl)-1*H*-pyrazol-3-yl]-*N*-(2-fluoroethyl) pyridin-2-amine | 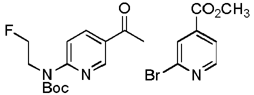 | A,B/10 |
| M269 | 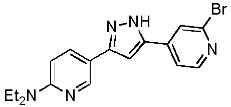 | 5-[5-(2-bromopyridin-4-yl)-1*H*-pyrazol-3-yl]-*N,N*-diethylpyridin-2-amine | 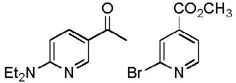 | A/47 |
| M276 | 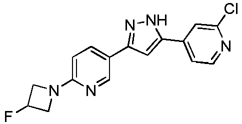 | 2-chloro-4-{3-[6-(3-fluoroazetidin-1-yl)pyridin-3-yl]-1*H-*pyrazol-5-yl}pyridine | 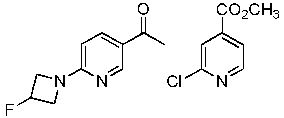 | A/52 |
| M277 | 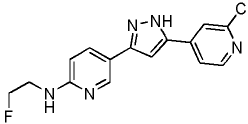 | 5-[5-(2-chloropyridin-4-yl)-1*H*-pyrazol-3-yl]-*N-*(2-fluoroethyl)-pyridin-2-amine | 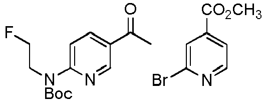 | E/28 |
| M279 | 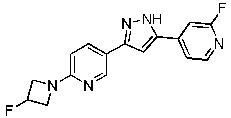 | 2-fluoro-4-{3-[6-(3-fluoroazetidin-1-yl)pyridin-3-yl]-1*H-*pyrazol-5-yl}pyridine | 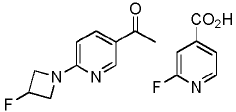 | C/48 |
| M282 | 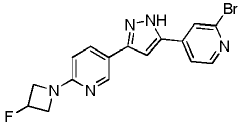 | 2-bromo-4-{3-[6-(3-fluorazetidin-1-yl)pyridin-3-yl]-1*H-*pyrazol-5-yl}pyridine | 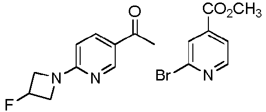 | A/83 |
| M286 | 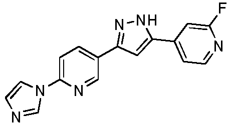 | 2-fluoro-4-{3-[6-(1*H*-imidazol-1-yl)pyridin-3-yl]-1*H-*pyrazol-5-yl}pyridine | 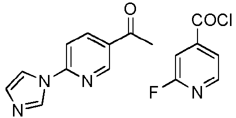 | D/41 |
| M287 | 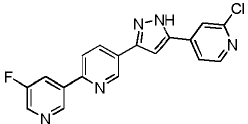 | 5-[5-(2-chloro pyridin-4-yl)-1*H-*pyrazol-3-yl]-5'-fluoro-2,3'-bipyridine | 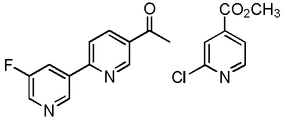 | A/45 |

**Table 2**

| **No.** | **HPLC Retention Time (min)** | **Mass Spec [M+H]⁺** | **¹H-NMR Data (ppm) δ (solvent, T)** |
|---|---|---|---|
| M268 | 15.1^{a} | 362.2 (100%), 364.2 (98%) | 13.54 (bs, 1H), 8.45 (d, *J* = 2.0 Hz, 1H), 8.41 (d, *J* = 5.0 Hz, 1H), 8.01 (s, 1H), 7.84 (d, *J* = 5.0 Hz, 1H), 7.78 (bd, *J =* 8.2 Hz, 1H), 7.28 (bs, 1H), 7.10 (bs, 1H), 6.63 (d, *J =* 8.7 Hz, 1H), 4.61 (t, *J* = 5.1 Hz, 1H), 4.50 (t, *J* = 5.1 Hz, 1H), 3.65 (q, *J* = 5.3 Hz, 1H), 3.59 (q, *J* = 5.3 Hz, 1H) (DMSO-d6, 298K) |
| M269 | 17.2^{a} | 372.3 (100%), 374.3 (98%) | 8.44 (d, *J* = 5.2 Hz, 1H), 8.40 (bs, 1H), 8.31 (bd, *J* = 9.4 Hz, 1H), 8.06 (s, 1H), 7.87 (dd, *J* = 5.2, 1.3 Hz, 1H), 7.57 (s, 1H), 7.33 (bd, *J* = 9.4 Hz, 1H), 3.69 (q, *J* = 7.0 Hz, 4H), 1.20 (t, *J* = 7.0 Hz, 6H) (DMSO-d6, 298K) |
| M276 | 14.6^{a} | 330.2 (100%), 332.2 (35%) | 13.63 (bs, 1H), 8.55 (s. 1H), 8.45 (d, *J* = 5.0 Hz, 1H), 7.94 (bd, *J* = 7.9 Hz, 1H), 7.89 (s, 1H), 7.82 (d, *J* = 5.0 Hz, 1H), 7.36 (bs, 1H), 6.58 (d, *J* = 8.7 Hz, 1H), 5.53 (dsept, *J* = 57.5, 2.9 Hz, 1H), 4.38-4.28 (m, 2H), 4.10-4.01 (m, 2H) (DMSO-d6, 298K) |
| M277 | 14.1^{a} | 318.1 | 9.35-8.98 (bs, 1H), 8.48 (d, *J* = 5.2 Hz, 1H), 8.36-8.25 (m, 2H), 7.89 (s, 1H), 7.79 (dd, *J* = 5.2, 1.3 Hz, 1H), 7.53 (s, 1H), 7.24 (d, *J* = 9.9 Hz, 1H), 4.66 (dt, *J* = 47.2, 4.8 Hz, 2H), 3.75 (dt, *J* = 27.7, 4.8 Hz, 2H) (DMSO-d6 + 2% TFA-d1) |
| M279 | 13.9^{a} | 314.3 (100%) | 13.63 (bs, 1H), 8.55 (d, *J* = 1.6 Hz, 1H), 8.28 (d, *J* = 4.7 Hz, 1H), 7.94 (d, *J* = 7.8 Hz, 1H), 7.77 (d, *J* = 4.7 Hz, 1H), 7.53 (s, 1H), 7.34 (bs, 1H), 6.58 (d, *J* = 8.6 Hz, 1H), 5.53 (dsept, *J* = 57.5, 2.9 Hz, 1H), 4.38-4.28 (m, 2H), 4.10-4.01 (m, 2H) (DMSO-d6, 298K) |
| M282 | 15.0^{a} | 374.2 (100%), 376.1 (98%) | 13.62 (bs, 1H), 8.55 (d, *J* = 2.1 Hz, 1H), 8.42 (d, *J* = 5.1 Hz, 1H), 8.02 (s, 1H), 7.94 (dd, *J* = 8.6, 2.1 Hz, 1H), 7.85 (dd, *J* = 5.1, 1.1 Hz, 1H), 7.37 (s, 1H), 6.58 (d, *J* = 8.6 Hz, 1H), 5.52 (dsept, *J* = 57.4, 2.9 Hz, 1H), 4.37-4.28 (m, 2H), 4.10-4.01 (m, 2H) (DMSO-d6, 298K) |
| M286 | 13.9^{a} | 307.3 (100%) | 8.95 (d, *J* = 1.9 Hz, 1H), 8.59 (s, 1H), 8.39 (dd, *J* = 8.5, 2.2 Hz, 1H), 8.33 (d, *J* = 5.2 Hz, 1H), 8.01 (s, 1H), 7.97 (d, *J* = 8.6 Hz, 1H), 7.79 (bd, *J* = 5.0 Hz, 1H), 7.64 (s, 1H), 7.57 (s, 1H), 7.16 (s, 1H) (DMSO-d6 + 1% DCI, 298K) |
| M287 | 11.8^{b} | 352.4 (100%), 354.2 (35%) | 9.23 (s, 1H), 9.18 (d, *J* = 1.8 Hz, 1H), 8.66 (d, *J* = 2.7 Hz, 1H), 8.49 (d, *J* = 5.2 Hz, 1H), 8.39 (dt, *J* = 10.3, 2.2 Hz, 1H), 8.33 (dd, *J* = 5.2, 2.2 Hz, 1H), 8.27 (d, *J* = 8.3 Hz, 1H), 7.92 (s, 1H), 7.84 (dd, *J* = 5.2, 1.3 Hz, 1H), 7.70 (s, 1H) (DMSO-d6, 298K) |

| | | | |
|---|---|---|---|
| ^{a} gradient 5% CH₃CN /95% H₂O → 100% CH₃CN in 30 min ^{b} gradient 50% CH₃CN /50% H₂O → 100% CH₃CN in 30 min | | | |

### Example 2: Binding studies

### Preparation of fibrils

Alpha-Synuclein was purified from *E*. *coli* according to established protocols (Nuscher B, et al., J. Biol. Chem., 2004; 279(21): 21966-75) and fibrils were prepared by aggregation of recombinant protein via constant agitation. In detail, 70 µM of alpha-synuclein mixed with 100 mM NaCl in 50 mM Tris, pH=7.0 + 0.02% NaN₃ were incubated at 1,400 rpm, 37°C for 96 h (Deeg AA, et al., Biochim. Biophys. Acta, 2015;1850(9): 1884-90). Secondary to aggregation, the resulting fibrils were sonicated free-floating in an ultrasonic bath for 10 min at room temperature. Fibril formation was verified using Thioflavin (Th)T fluorescence and sucrose density centrifugation. ThT fluorescence was measured using a LS55 Luminescence Spectrometer (Perkin Elmer, Hamburg, Germany) with 0.5 µM alpha-synuclein and 20 µM ThT in 50 mM Tris*HCl, pH 7.5 (Kuebler L, et al., Eur. J. Nucl. Med. Mol. Imaging, 2021 Jun; 48(6): 1759-1772).

### Competition binding assay

Competition binding assays were performed according to established protocols with minor modifications (Saw RS, et al., preprint. 2024: https://doi.org/10.21203/rs.3.rs-2189800/v1). In detail, fibrils were incubated with 1 nM [³H]MODAG-005 and a 1sqrt(10) (= 1:3.16) serial dilutions of the non-radioactive compound of interest starting from 1 µM diluted in a reaction buffer composed of 30 mM Tris*HCl with 10% pure ethanol and 0.05% Tween20 at pH 7.4.

96-well plates were incubated under agitation at 37°C, 45 rpm for 2 h covered by plastic film (Resealable tape, Revvity, Hopkinton, MA, U.S). Prior to harvesting, a filtermat (Revvity, Hopkinton, MA, U.S.) was incubated with 1 % (v/v) polyethylenimine (PEI, Sigma Aldrich Chemie GmbH, Taufkirchen, Germany) at 4 °C for 30 min. After incubation, excessive PEI was removed by washing the filter with reaction buffer and bound and free ligands were separated by vacuum filtration using a harvester (MicroBeta^{®} Filtermat-96 Cell Harvester, Revvity, Hopkinton, MA, U.S.). The filter was washed three times with ice-cold reaction buffer and subsequently dried in a microwave oven for 2 min at medium power. After covering with a MeltiLex^{®} Scintillator Plate (Revvity, Hopkinton, MA, U.S) at 120 °C, the filtermats were sealed in a plastic bag (Sample bag for MicroBeta^{®}, Revvity, Hopkinton, MA, U.S.) and read in a MicroBeta² Microplate Counter for Radiometric and Luminescence Detection (Revvity, Hopkinton, MA, U.S.). Luminescence counting was performed for two minutes per well. Radioactivity was plotted against the increasing tritium-labeled compound or cold compound concentrations. Data points were fitted using nonlinear regression analysis in GraphPad Prism (GraphPad Software, LLC., Version 10.1.1, Boston, MA, USA). Preferably, the resultant affinity Ki value should be at most 9.8 nM, which is the Ki value observed for emrusolmin (data shown in WO 2021/099518).

### Example 3: Metabolic stability determination

### Metabolic stability of compounds in human liver microsomes

The compounds were incubated with liver microsomes, and collected samples were analyzed by UPLC/HR-MS to measure stability of the compounds and to predict *in vivo* clearance. In particular, the compounds were incubated with liver microsomes (mixed gender) under the following specifications:
Time points: 0, 10, 20, 40, 60 min ± cofactors; concentration of the tested compound: 1 µM; protein content: 0.5 mg/ml; replicates: 2 with cofactors, 1 without; buffer: 0.1 M phosphate buffer pH 7.4, 5 mM MgCl₂; cofactors: NADPH (1 mM), UDPGA (2 mM), alamethicin (10 µg/ml); incubation volume: 300 µl; sampling volume: 40 µl; spiking solvent: 20% DMSO (1/100 to incubation, if allowed by solubility); quenching solvent: 2-fold volume of 75% CAN. As control was used midazolam (1 µM) disappearance rate & blank incubation without study compound. The collected samples were stored at -20°C until thawed, centrifuged and analyzed.

The samples were analyzed by UPLC/HR-MS (with data dependent MS/MS mode) to monitor substrate depletion. The analytical method was optimized by using the parent compound for optimum chromatographic properties (peak shape and retention) and mass spectrometric ionization. Disappearance rate of the study compound was estimated based on relative LC/MS peak areas (0 min marked as 100%) and was used to calculate *in vitro* half-life and clearance (and *in vivo* extrapolation of hepatic clearance). A clearance value ≤ 100 µl/min/mg can be considered in the desired range.

### Example 4: Induction of CYP1A2

### Induction of CYP enzyme 1A2 in human cryopreserved pooled hepatocytes

Induction potency compounds towards human CYP enzyme 1A2 (mRNA level) was studied in cryopreserved hepatocytes (pooled donor lot) cells at three concentrations.

In particular, the test compounds were dissolved in DMSO and stock solutions at 200-fold the test concentrations were prepared in DMSO. DMSO stock solutions were spiked in cell culture media shortly before the start of incubations or medium replacement (after 24 hours). Aliquots frozen to -20°C were used in the second experimental day.

Cryopreserved human hepatocytes (one pooled donor lot) cells were plated and exposed to study compounds for 48h, replacing fresh medium approximately at 24 hours after start of incubation, after which the cells were lysed, and total RNA was isolated. Genomic DNA was digested, and cDNA was generated with M-MuLV reverse transcriptase (RT) enzyme. Target mRNA-levels (CYP1A2) were then analyzed by real-time quantitative PCR using TaqMan probe chemistry. Reference gene GAPDH was used as normalizing control. Possible toxicity of the study compounds towards the cells was monitored visually during the study.

Omeprazole at a concentration of 50 µM was used as positive control inducer of CYP1A2. CYP1A2 induction levels below 2-fold are considered as essentially no induction, induction levels up to 3-fold as low induction levels. Thus, preferably, the CYP1A2 induction level should be ≤ 3-fold, more preferably ≤ 2-fold.

**Table 3: Values for affinity for alpha-synuclein aggregates as depicted as Kᵢ against 1 nM [³H]MODAG-005 were obtained by the method described in Example 2. Values for clearance in human microsomes were obtained by the method described in Example 3. Values for CYP1A2 induction as depicted in fold induction at 1 µM were obtained by the method described in Example 4.**

| **No.** | **Structure** | **Affinity Kᵢ [nM]** | **Clearance (Human microsomes) [µl/min/mg]** | **CYP1A2 (Fold induction at 1 µM)** |
|---|---|---|---|---|
| M268 | 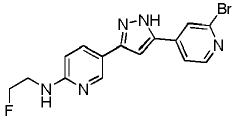 | 2.7 | 16.68 | 3.0 |
| M269 | 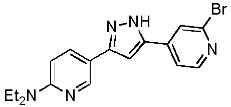 | 9.3 | 53.13 | 2.4 |
| M276 | 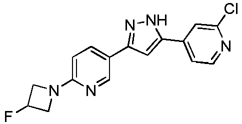 | 0.5 | 64.62 | 0.9 |
| M277 | 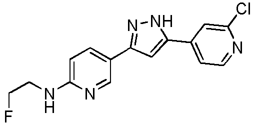 | 6.1 | 23.48 | 2.0 |
| M279 | 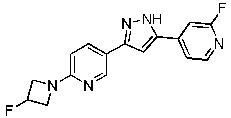 | 2.3 | 94.92 | 1.1 |
| M282 | 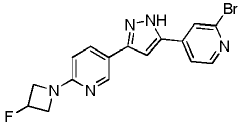 | 0.5 | 46.70 | 1.3 |
| M286 | 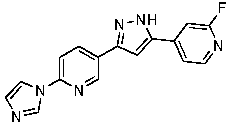 | 0.4 | N/A | N/A |
| M287 | 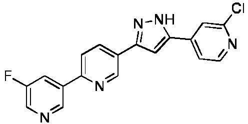 | 0.5 | N/A | N/A |

**Table 4 (Reference compounds of WO 2021/099518 ): Values for affinity for alpha-synuclein aggregates as depicted as Kᵢ against 1 nM [³H]MODAG-005 were obtained by the method described in Example 2. Values for clearance in human microsomes were obtained by the method described in Example 3. Values for CYP1A2 induction as depicted in fold induction at 1 µM were obtained by the method described in Example 4.**

| **No.** | **Structure** | **Affinity Kᵢ [nM]** | **Clearance (Human microsomes) [µl/min/mg]** | **CYP1A2 (Fold induction at 1 µM)** |
|---|---|---|---|---|
| M204 | 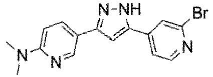 | 0.7 | 130.98 | 2.3 |
| M225 | 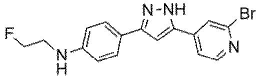 | 0.7 | 16.46 | 4.1 |
| M255 | 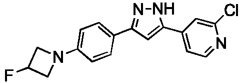 | 1.2 | 115.85 | 2.6 |

All inventive compounds shown in Table 3 exhibit the desired properties of Ki ≤ 9.8 nM (which is the Ki of emrusolmin), clearance ≤ 100 µl/min/mg and CYP1A2 induction ≤ 3-fold. Thus, they provide a combination of good pharmaceutical activity, metabolic stability and low AhR activation. The reference compounds shown in Table 4, on the other hand, fail in at least one respect and are thus less advantageous as pharmaceutical compounds.

## Claims

1. A compound represented by the general formula la or Ib 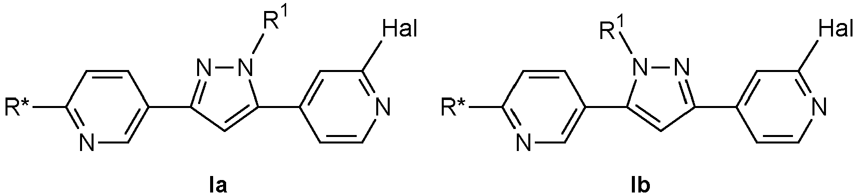 or a prodrug, solvate or pharmaceutically acceptable salt thereof,
wherein
R* is selected from 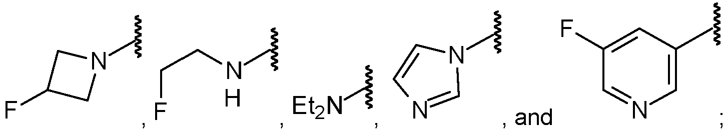
R¹ is independently selected from hydrogen, C₁₋₄ alkyl and -(CH₂)-O-P(=O)(OR)(OR),
wherein C₁₋₄ alkyl can be optionally substituted by one or more halogen;
R is hydrogen or a cation; and
Hal is halogen.

2. The compound according to claim 1, wherein R* is selected from

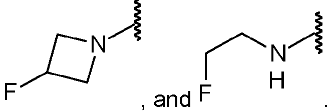

3. The compound according to claim 1 or 2, wherein R¹ is hydrogen.

4. The compound according to any one of claims 1 to 3, wherein R is hydrogen.

5. The compound according to any one of claims 1 to 4, wherein Hal is selected from Br, Cl and F, preferably wherein Hal is Cl.

6. A pharmaceutical composition comprising a compound as defined in any one of claims 1 to 5 or a prodrug, solvate or pharmaceutically acceptable salt thereof and optionally a pharmaceutically acceptable carrier.

7. A compound according to any one of claims 1 to 5 or a prodrug, solvate or pharmaceutically acceptable salt thereof for use in therapy.

8. A compound according to any one of claims 1 to 5 or a prodrug, solvate or pharmaceutically acceptable salt thereof for use in the treatment of a disease linked to alpha-synuclein aggregation.

9. The compound for use according to claim 8, wherein the disease linked to alpha-synuclein aggregation is selected from Parkinson's disease, dementia with Lewy bodies, multiple system atrophy, Alzheimer's disease, progressive supranuclear palsy, and frontotemporal dementia.

10. Use of a compound according to any one of claims 1 to 5 or a prodrug, solvate or pharmaceutically acceptable salt thereof for inhibiting alpha-synuclein aggregation *in vitro* or *ex vivo.*
